# FASCICULE DE BREVET EUROPEEN

(11) **EP 2 349 014 B1**
(45) Date de publication et mention de la délivrance du brevet: **06.07.2016**
(21) Numéro de dépôt: 09807925.4
(22) Date de dépôt: 14.08.2009
(51) Int. Cl.: A61B 17/00, A61M 25/01, G01L 1/22, A61B 90/00

(54) **DISPOSITIF D'INTERVENTION CHIRURGICALE COMPRENANT UNE AIGUILLE SUSCEPTIBLE DE SE DÉFORMER**
VORRICHTUNG FÜR CHIRURGISCHE VERFAHREN MIT EINER VERFORMBAREN NADEL
SURGICAL PROCEDURE DEVICE COMPRISING A NEEDLE CAPABLE OF DEFORMING

(30) Priorité: 19.08.2008 FR 0855617
(43) Date de publication de la demande: 03.08.2011
(73) Titulaire: Université Grenoble Alpes, 38400 Saint-Martin-d'Hères (FR)
(72) Inventeur: MOREAU-GAUDRY, Alexandre, 38240 Meylan (FR); BONVILAIN, Agnes, 73800 Myans (FR)
(74) Mandataire: Regimbeau
(86) Numéro de dépôt international: PCT/EP2009/060561
(87) Numéro de publication internationale: WO 2010/020591

(56) Documents cités:
- EP-A- 1 857 038
- WO-A-94/19051
- WO-A-99/60267
- DE-A1- 4 408 730
- US-A1- 2003 236 445

## Description

### DOMAINE DE L'INVENTION

La présente invention concerne un dispositif d'intervention chirurgicale comprenant une aiguille chirurgicale apte à traverser des tissus humains ou animaux susceptible de se déformer et un système de détermination de la position de l'aiguille et de la position de son extrémité distale, c'est-à-dire l'extrémité destinée à intervenir sur une cible déterminée, par rapport à son extrémité proximale, c'est-à-dire l'extrémité reliée à un support intermédiaire manipulable par le praticien.

### ARRIERE PLAN DE L'INVENTION

Dans le cadre de procédures médicales percutanées à visée diagnostique ou thérapeutique, guidées ou non par une imagerie, un instrument susceptible de se déformer est introduit à travers la peau pour atteindre une cible préalablement identifiée.

Différents types d'instruments sont communément utilisés dans la pratique médicale quotidienne pour de telles interventions, comme, par exemple, des sondes, des guides de cathéters, des cathéters, des fibroscopes, des palpeurs, des tiges, des aiguilles.

Pour faciliter la réalisation de ces procédures, des outils de localisation et de navigation ont été développés permettant de visualiser, en temps réel et dans un environnement virtuel représentatif de la réalité, la cible visée, la position du modèle de l'instrument ainsi que sa trajectoire prévisionnelle.

L'aide apportée au clinicien par ces nouveaux outils lui permet de guider l'instrument vers la cible de manière plus précise avec une diminution de la morbidité.

Néanmoins, les outils de localisation et de navigation actuels font l'hypothèse de l'indéformabilité de l'instrument utilisé, hypothèse souvent non vérifiée dans la pratique courante du fait des interactions de l'instrument avec le tissu humain ou animal.

En effet, l'interaction de l'instrument déformable avec le tissu humain ou animal (tissu mou, obstacle dur osseux, ou autre) est à l'origine de déformations de l'instrument qui peuvent entraîner l'échec de la procédure médicale interventionnelle.

Par exemple, l'interaction du biseau d'une aiguille droite flexible avec le tissu lors d'une ponction-biopsie profonde peut générer une déflexion responsable de l'échec de la ponction-biopsie, avec lésions potentielles de tissus adjacents (nerfs, artères).

Inversement, la cible peut être mobilisée suite à l'interaction de l'instrument avec le tissu humain mou à l'origine d'un échec de la procédure.

Face à ces difficultés, il apparaît donc nécessaire d'enrichir les environnements virtuels de navigation et représentatifs de la réalité, en leur conférant la capacité à suivre la position exacte de l'ensemble de l'instrument déformable et déformé afin de pouvoir préciser, en temps réel, les positions relatives de l'instrument, de son extrémité distale et de la cible visée.

Il serait également souhaitable de pouvoir contraindre localement l'instrument déformable pour corriger sa trajectoire en vue d'atteindre la cible visée et d'améliorer la qualité de la procédure médicale.

Ainsi, le document US 2005/0059883, qui est considéré l'état de la technique le plus proche de la présente invention, décrit le positionnement de jauges de contraintes sur la partie proximale d'une aiguille flexible, en vue de détecter la déflexion de l'aiguille. La valeur de cette déflexion est prise en compte par le système de navigation pour indiquer la position de l'extrémité distale de l'aiguille. Toutefois, le type de dispositif décrit ne permet de ne rendre compte que d'une déformation simple (déflexion) et non des déformations complexes (multiples courbures), pourtant plus représentatives de la réalité, en raison de l'inhomogénéité des forces appliquées le long de l'aiguille. En outre, déterminer la position de l'extrémité de l'aiguille à partir de données de déformation proximale sous-entend une certaine « régularité » de déformation de l'aiguille, pouvant, en particulier, être incompatible avec la nature même de l'aiguille (multiples courbures). Enfin, le dispositif proposé est passif, c'est-à-dire qu'il ne permet pas de modifier la trajectoire de l'outil.

Les documents DE 44 08 730 et WO 94/19051 concernent des instruments souples destinés à être introduits dans des cavités naturelles et non adaptés pour traverser des tissus corporels.

Le brevet US 5,830,144 prévoit d'entourer l'outil d'une gaine élastomérique ou rigide en vue de suivre sa position en temps réel. La gaine contient des éléments piézo-électriques fournissant un signal de détection de la position de l'instrument par un système de localisation échographique ou électromagnétique. Toutefois, les déformations de l'instrument ne sont pas déterminées de manière intrinsèque à l'instrument mais de manière extrinsèque : la gaine, élément externe à l'instrument, doit en effet être visible en temps réel par le système de localisation pour pouvoir identifier ses déformations, à partir desquelles sont déduites celles de l'instrument contenu dans la gaine. La nécessité de la visibilité de la gaine au sein du tissu humain constitue une limitation importante du dispositif présenté.

En outre, la qualité de la jonction entre la gaine et l'instrument apparaît comme essentielle pour pouvoir déduire la position de l'instrument à partir de la position de la gaine.

En effet, un instrument destiné à traverser des tissus présente naturellement une surface particulièrement lisse, mais cet état de surface peut ne pas permettre d'assurer une adhérence correcte d'un élément rapporté tel qu'une gaine. Il peut donc se produire un déplacement des capteurs par rapport à l'instrument, induisant des imprécisions de mesure, avec des conséquences potentiellement dramatiques pour la réalisation de gestes nécessitant une grande précision.

Le document US 7,261,686 propose l'utilisation d'un guide de cathéter comprenant une pluralité d'actionneurs disposés sur sa longueur et une unité de contrôle de ces actionneurs recevant des informations, par exemple, de jauges de contraintes. Dans le dispositif proposé, le guide de cathéter est introduit dans une structure anatomique creuse et a pour objet de se déformer afin de permettre au cathéter de se diriger dans la direction souhaitée. Une fois mis en position, il peut être « ancré » par modification de sa rigidité. Très utile pour la mise en place de cathéter, ce dispositif nécessite le contrôle d'une imagerie directe de la partie au sein de l'organisme. En outre, il ne permet qu'un guidage indirect, par l'intermédiaire du guide, de l'instrument (le cathéter), et non la navigation directe de l'instrument inséré dans le guide.

Le document US 2007/0016067 présente un dispositif robotisé permettant de guider une aiguille biseautée vers une cible par la combinaison de mouvements de translation et rotation de cette dernière. Cette technique nécessite la modélisation des propriétés mécaniques tissulaires ainsi que la détection de l'aiguille et de son extrémité sur une imagerie acquise de manière périodique. L'utilisation d'un modèle cinématique non holonomique associée à une combinaison judicieuse des paramètres de translation et de rotation de l'aiguille permet de corriger la trajectoire de l'aiguille. Toutefois, la mise en oeuvre de cette méthode nécessite d'être capable de détecter l'aiguille et son extrémité, processus aisé pour des imageries rayons X (images fluoroscopiques) mais pouvant être beaucoup plus difficile pour des dispositifs d'imagerie ultrasonore, par exemple (l'identification de l'extrémité d'une aiguille constitue d'ailleurs une des difficultés des ponctions guidées sous imagerie échographique). En outre, pour une imagerie échographique 2D, cela implique implicitement d'avoir un positionnement de l'aiguille dans le plan d'acquisition image.

Dans «'Smart' Needle for Percutaneous Surgery: Influencial Factor Investigation», de Yan et al (Proceedings of the 29th Annual international Conference of the IEEE EMBS, 23-26/08/2007), les auteurs s'intéressent à la connaissance de la déflection d'une aiguille et de sa mobilisation par l'utilisation d'actionneurs piézo-électriques. Dans l'approche présentée, la détection de l'extrémité de l'aiguille est réalisée par l'utilisation d'un capteur électromagnétique disposé à l'extrémité de l'aiguille. Dans ce cas, il est toutefois nécessaire de disposer d'une visualisation directe de l'aiguille pour agir de manière appropriée. Il faut en outre signaler les limitations actuelles des systèmes électromagnétiques de localisation.

Un premier but de l'invention est de proposer un dispositif capable de prendre en compte les déformations complexes, et a fortiori les déformations simples, d'une aiguille destinée à traverser les tissus humains ou animaux.

Un tel dispositif devra permettre de connaître, à tout moment, au moyen d'un système de localisation ou de navigation, la position de l'extrémité distale de l'aiguille par rapport à sa partie proximale, et/ou la position de l'ensemble de l'aiguille potentiellement déformé par rapport à sa partie proximale.

Un autre but de l'invention est de contraindre localement l'aiguille déformable en vue de faciliter son guidage vers la cible visée.

### BREVE DESCRIPTION DE L'INVENTION

Conformément à l'invention, il est proposé un dispositif d'intervention chirurgicale, comprenant une aiguille chirurgicale apte à traverser des tissus humains ou animaux et un système comprenant des composants dits « passifs » aptes à mesurer une déformation ou une contrainte locale de l'aiguille, ledit système comprenant au moins deux séries de composants passifs agencés à la surface de l'aiguille de manière à établir une relation biunivoque entre la position de l'aiguille ou la position de l'extrémité distale de l'aiguille et l'ensemble des données issues des dites séries.

Selon un premier mode de réalisation, ledit système comprend au moins deux séries de composants passifs disposés selon deux génératrices de la surface de l'aiguille appartenant à deux plans non confondus passant par l'axe de l'aiguille, définissant ainsi un référentiel dans un plan orthogonal à l'axe de l'aiguille.

Lesdits composants passifs comprennent avantageusement des microsystèmes électromécaniques, tels que des capteurs piézoélectriques et/ou des jauges de contraintes.

De façon préférée, les composants passifs sont incorporés à la surface de l'aiguille.

Selon un mode particulier de réalisation, chaque série comprend un unique composant passif, lequel comporte un élément conducteur longitudinal, se présentant sous la forme d'au moins une spire, s'étendant parallèlement à l'axe de l'aiguille sur sensiblement toute la longueur de l'aiguille.

L'aiguille présente de préférence au moins une zone déformable, telle qu'une diminution locale d'épaisseur et/ou une articulation, et les composants passifs sont agencés sur ladite zone.

Selon un autre aspect, ledit système comprend en outre au moins deux séries de composants actifs agencés sur au moins une zone de déformation préférentielle de l'aiguille.

Lesdits composants actifs comprennent avantageusement des microsystèmes électromécaniques, tels que des actionneurs thermiques, piézoélectriques, pneumatiques, électromagnétiques, et/ou électrostatiques.

De préférence, les composants actifs sont incorporés à la surface de l'aiguille.

Selon un mode de réalisation particulièrement avantageux, ledit système comprend au moins deux séries de composants passifs et deux séries de composants actifs.

Par exemple, les composants passifs de chaque série sont répartis sensiblement sur toute la longueur de l'aiguille chirurgicale et/ou les composants actifs sont agencés sur au moins une zone de déformation préférentielle de la partie distale de l'aiguille.

Le dispositif comprend de préférence un système de couplage des composants passifs et actifs.

Un autre objet concerne un procédé de détermination de la position de l'extrémité distale d'une aiguille chirurgicale apte à traverser des tissus humains ou animaux, comprenant l'agencement, à la surface de l'aiguille, d'au moins deux séries de composants dits « passifs » aptes à mesurer une déformation ou une contrainte locale de l'aiguille, et une calibration dans laquelle on établit une relation biunivoque entre l'ensemble des données issues de chaque série de composants passifs et la position de l'aiguille ou la position de l'extrémité distale de l'aiguille, et, le cas échéant, l'agencement, à la surface de l'aiguille, d'au moins deux séries de composants dits « actifs » aptes à imposer une contrainte locale à l'aiguille.

Ledit procédé comprend en outre une étape de localisation de la cible à atteindre et de l'extrémité distale de l'aiguille.

Un autre objet de l'invention concerne enfin un procédé de fabrication d'un dispositif d'intervention chirurgicale tel que décrit plus haut, comprenant l'incorporation, sur la surface de l'aiguille, des composants passifs et, le cas échéant, des composants actifs, au moyen de techniques de micro-fabrication.

### BREVE DESCRIPTION DES DESSINS

D'autres caractéristiques et avantages de l'invention ressortiront de la description détaillée qui va suivre, en référence aux dessins annexés sur lesquels
- la figure 1 illustre différentes déformations possibles d'une aiguille et les référentiels associés à la base de l'aiguille et au localisateur ;
- les figures 2A à 2C illustrent de manière schématique la structure et le principe de fonctionnement d'une jauge de contraintes ;
- les figures 3A et 3B illustrent un exemple de disposition de deux séries de composants passifs sur une aiguille biseautée, en vue de face et en vue de côté, non déformé (fig. 3A) et déformé (fig. 3B) ;
- la figure 4 représente la connexion en série de deux composants passifs fixés sur la surface de l'aiguille ;
- la figure 5 est un schéma d'un dispositif électronique permettant de traiter les informations issues des composants passifs;
- la figure 6 illustre un mode de réalisation particulier de l'incorporation de composants passifs à la surface de l'aiguille;
- la figure 7 illustre la connexion en série de deux composants passifs incorporés à la surface de l'aiguille;
- la figure 8 schématise un actionneur multicouche pouvant être utilisé comme composant actif ;
- la figure 9 illustre l'incorporation à la surface de l'aiguille de micro-articulations comprenant des actionneurs thermiques ;
- la figure 10 présente un mode de réalisation préféré de l'invention, associant deux séries de composants passifs et deux séries de composants actifs incorporés directement dans l'épaisseur de l'aiguille.
la figure 11 présente un mode de réalisation avantageux de l'invention dans lequel deux séries de composants passifs sont incorporées sur toute la longueur de l'aiguille et deux séries de composants actifs sont incorporées dans la région distale de l'extrémité distale de l'aiguille.

### DESCRIPTION DETAILLEE DE L'INVENTION

Le dispositif comprend une aiguille à laquelle est associé un système passif apte à mesurer les déformations de l'aiguille et, éventuellement, un système actif apte à appliquer des déformations à l'aiguille chirurgical.

L'aiguille à laquelle s'applique l'invention est un instrument apte à traverser les tissus, à la différence d'un instrument destiné à emprunter des canaux anatomiques. Elle n'est toutefois pas parfaitement rigide et est susceptible de se déformer.

L'aiguille peut être employée par exemple pour une ponction, pour une biopsie ou pour délivrer au niveau d'une cible anatomique une thérapeutique (par exemple, un antibiotique, un anti-inflammatoire).

L'aiguille considérée présente une forme allongée, c'est-à-dire que sa longueur est bien supérieure à son diamètre. De manière préférée, l'aiguille sera de topologie cylindrique.

L'aiguille est typiquement dans un matériau métallique, mais peut aussi être fabriquée en tout matériau biocompatible et compatible avec l'utilisation des composants passifs et/ou actifs décrits plus bas.

On définit pour cette aiguille une extrémité proximale, ou base, qui est l'extrémité reliée au dispositif qui permet au praticien de manipuler l'aiguille, et une extrémité distale opposée, qui est destinée à atteindre la cible visée.

Pour faciliter la pénétration dans les tissus, l'extrémité distale est de préférence pointue, par exemple en forme de biseau.

S'il s'agit d'une aiguille destinée à une ponction ou à la délivrance d'une substance thérapeutique (antibiotique, anti-inflammatoire), elle est creuse.

Ainsi, dans le cas d'une aiguille destinée à des ponctions ou des biopsies profondes, le diamètre extérieur est de l'ordre de 1 à 1,8 mm, l'épaisseur de la paroi est d'environ 0,3 mm, la longueur d'une dizaine de cm (par exemple 17cm).

Ces éléments descriptifs sont des exemples qui sont naturellement variables en fonction de la profondeur de la cible et de la nature du tissu à traverser pour atteindre la cible.

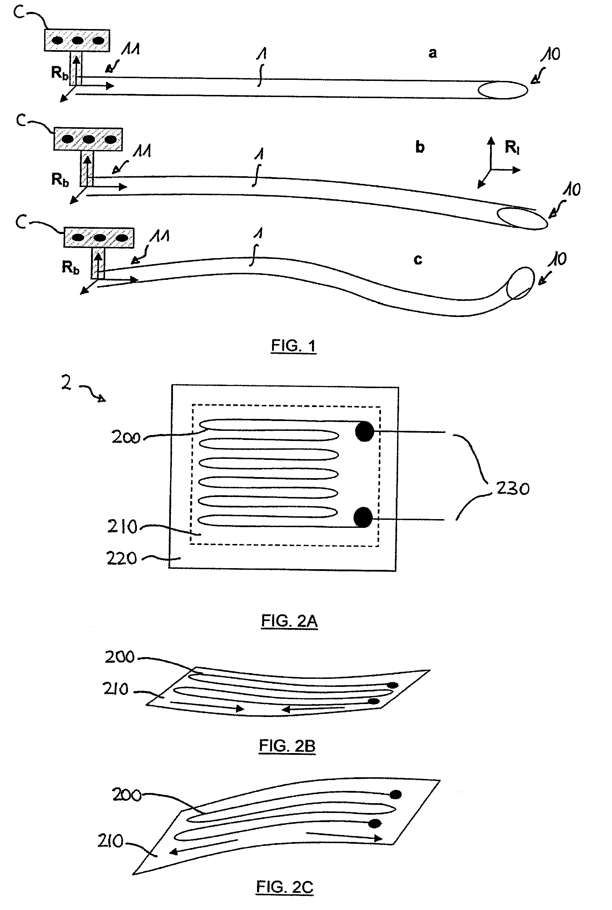

La figure 1 représente schématiquement l'aiguille 1 dans une position non déformée (schéma a) et deux positions déformées, l'une consistant en une déformation simple ou déflexion (schéma b), l'autre en une déformation complexe, à courbures multiples (schéma c).

Le dispositif conforme à l'invention est conçu de manière à restituer avec précision les informations relatives à ces différents types de déformations.

Sont maintenant décrits les systèmes passif et actif associés à l'aiguille.

### Composants passifs

Le système passif est constitué d'un système mécanique comprenant des composants passifs et d'un système électronique apte à traiter les informations issues des composants.

Les composants passifs sont des composants aptes à mesurer une contrainte ou une déformation locale et à la transformer en un signal électrique (tension, intensité...) caractéristique de cette déformation.

Chaque composant est fixé sur une région de faible superficie (typiquement inférieure à 10 mm²) de la surface de l'aiguille, et mesure localement la déformation de la surface sur laquelle le composant est fixé, ou la contrainte exercée sur cette surface.

Les composants passifs sont associés selon au moins deux séries.

Par série, on entend ici une entité dont on mesure un signal électrique représentatif d'une déformation de l'aiguille. On précise que dans le présent texte, la déformation de l'aiguille doit être comprise comme signifiant la déformation de l'axe de l'aiguille.

Si la série est constituée d'un unique composant passif, on prend en compte les signaux électriques en provenance de cet unique composant, ce qui donne une information sur la déformation ou la contrainte locale (à l'endroit de ce composant) ; si la série est constituée d'au moins deux composants judicieusement positionnés, on prend en compte l'ensemble des signaux électriques en provenance des différents composants et on obtient ainsi une information globale sur la déformation de l'aiguille.

Au moins deux séries distinctes de composants passifs étant disposés sur l'aiguille, la prise en compte de l'ensemble des signaux électriques en provenance des différentes séries de composants permet de déterminer l'ensemble des contraintes locales exercées sur l'aiguille.

On peut alors en déduire la contrainte globale et donc la déformation et la position de l'extrémité distale de l'aiguille par rapport à son extrémité proximale.

Différents types de composants passifs peuvent être utilisés, comme par exemple des microsystèmes électromécaniques (MEMS), et plus particulièrement des capteurs à base de matériaux piézo-électriques, des jauges de contrainte et/ou tout autre type de capteurs.

A titre d'exemple, les composants passifs choisis sont des jauges de contraintes, telles que celles commercialisées sous la référence FLK-2-17 par la société Tokyo Sokki Kenkyujo, qui sont spécifiquement adaptées aux mesures de contraintes sur des cylindres.

La structure et le principe de fonctionnement de ces jauges de contraintes sont exposés de manière schématique aux figures 2A à 2C.

Comme on peut le voir sur la figure 2A, la jauge de contrainte 2 comprend un long élément conducteur 200, qui est arrangé sous forme de spires sur le corps d'épreuve 210.

De manière générale, le conducteur, non déformé, est caractérisé par sa résistance électrique R et sa longueur L.

Le corps d'épreuve subit la déformation à l'origine de la déformation du long conducteur. Lors de déformations du conducteur, sa variation de résistance ΔR est une fonction f de celle de sa longueur ΔL : ΔR = f(ΔL).

L'élément conducteur 200 est relié à chacune de ses extrémités à des connecteurs 230.

L'ensemble est fixé sur un support 220 qui assure la liaison entre l'aiguille déformable et le corps d'épreuve 210.

La figure 2B illustre une première déformation qui tend à mettre en compression le corps d'épreuve 210 et l'élément 200 (rapprochement des flèches). Dans ce cas, la résistance globale de la jauge diminue en raison du raccourcissement de la longueur de l'élément conducteur.

La figure 2C illustre une déformation inverse qui tend à mettre en tension le corps d'épreuve 210 et l'élément 200 (éloignement des flèches). Dans ce cas, la résistance globale de la jauge augmente en raison de l'augmentation de la longueur de l'élément conducteur.

Les jauges de contraintes et les capteurs piézo-électriques étant des composants répandus sur le marché, ils ne seront pas décrits plus en détail. L'homme du métier est à même de sélectionner dans les catalogues des différents fabricants les composants les plus appropriés en fonction de leur sensibilité, de leur capacité à être fixés sur l'aiguille, etc.

Il est bien sûr possible de combiner différents types de composants passifs sur la même aiguille, pour autant que les signaux électriques recueillis soient compatibles.

### Disposition des composants sur l'aiguille

Les composants d'une même série sont judicieusement positionnés sur la surface de l'aiguille et connectés pour atteindre l'objectif de l'invention.

Leur nombre et leur disposition sont choisis par l'homme du métier selon les possibilités de déformation de l'aiguille (par exemple, déflexion ou bien déformation à courbures multiples) et le but recherché.

D'une manière générale, on tient compte des possibilités qu'a l'aiguille de se déformer, notamment du fait de sa longueur et de son diamètre, et on positionne les composants aux lieux de concentration des contraintes, c'est-à-dire les lieux de déformation préférentielle de l'aiguille.

A cet effet, l'homme du métier peut effectuer une modélisation de l'aiguille et simuler sa déformation sous l'effet de contraintes prédéfinies en fonction de l'usage prévu. Pour donner des indications fiables, cette modélisation doit tenir compte de l'environnement de l'aiguille (nature des tissus traversés, interactions entre les tissus et l'aiguille...).

Pour éviter cette étape de détermination des lieux de concentration maximale des contraintes, on peut disposer les composants de chaque série sur toute la longueur de l'aiguille. On garantit ainsi une prise en compte de l'ensemble des déformations.

Ainsi, si l'aiguille est susceptible de se déformer selon des courbures multiples (cas d'une aiguille longue et fine), on répartit les composants sur sensiblement toute la longueur de l'aiguille de manière à mesurer plus finement les déformations locales à partir desquelles on déduira sa déformation globale.

L'intervalle entre deux capteurs est fonction des possibilités de déformation de l'aiguille et de la précision recherchée.

De manière particulièrement avantageuse, en vue d'éviter l'étape de modélisation des déformations de l'aiguille, on peut définir sur l'aiguille des zones déformables.

A cet effet, on peut par exemple créer des diminutions locales de l'épaisseur de l'aiguille, au niveau desquelles l'aiguille va fléchir préférentiellement.

De manière alternative, on peut utiliser une aiguille articulée, qui se déformera au niveau des articulations.

On impose ainsi les lieux de déformation préférentielle de l'aiguille, quelles que soient les contraintes qui s'y appliquent lors de la traversée des tissus.

Dans ce cas, on positionne les composants passifs sur les zones de déformation imposées.

Ainsi, et à titre d'exemple, pour une aiguille dont ont été identifiées (ou imposées) deux zones de déformation préférentielle, les capteurs seront positionnés à la surface de ces deux zones afin de prendre en compte au mieux les informations locales portées par ces zones de déformation, à partir desquelles pourra être déduite la position globale.

En revanche, si l'aiguille n'est susceptible que de se défléchir dans une direction privilégiée (cas d'une aiguille courte, par exemple), il peut être suffisant de placer les composants dans la région de l'extrémité proximale de l'aiguille.

Dans le cas où l'aiguille présente une forme cylindrique, on peut disposer deux séries de composants sur deux génératrices distinctes de la surface de l'aiguille.

Ces génératrices sont choisies de manière, d'une part, à prendre en compte les déformations préférentielles de l'aiguille, et d'autre part, à maximiser l'information acquise par chaque série de composants.

Chaque génératrice définit, avec l'axe de l'aiguille non déformée, un plan.

Dans un plan perpendiculaire à l'axe de l'aiguille, la trace des plans ainsi définis définit un référentiel et un système de coordonnées.

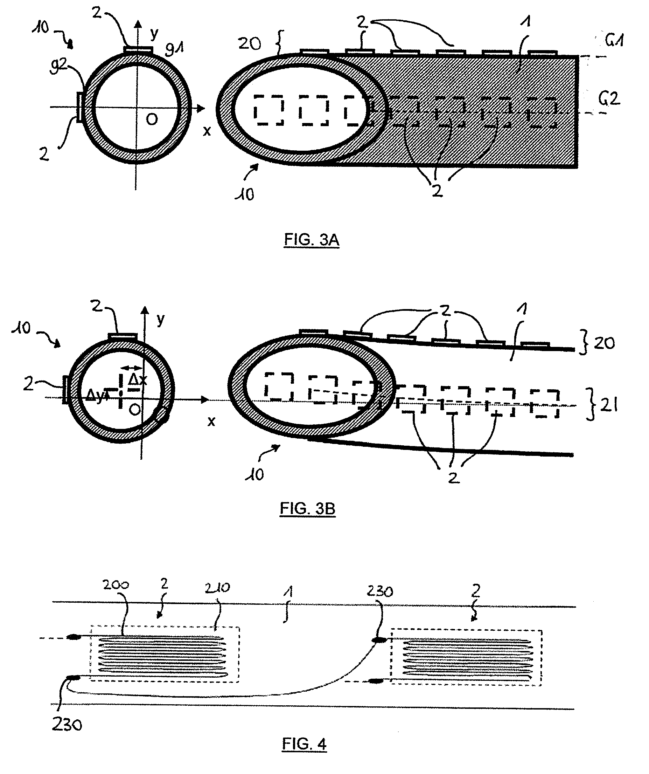

La figure 3A illustre un exemple de disposition de deux séries 20, 21 de composants passifs à la surface de l'aiguille 1.

Sur cette figure, les composants ont été disposés selon deux génératrices G1, G2 appartenant à deux plans orthogonaux.

Les traces de ces plans, visibles sur la partie gauche de la figure 3A, peuvent définir un référentiel orthogonal (O,x,y) de coordonnées cartésiennes ou polaires.

L'axe de l'aiguille non déformée possède donc des coordonnées (0, 0) dans ce référentiel.

La figure 3B illustre la même aiguille dans un état déformé. L'axe (ici, au niveau de l'extrémité distale 10) de l'aiguille possède maintenant des coordonnées (Δx, Δy) dans ce même référentiel.

Par ailleurs, il est avantageux, lorsque l'extrémité distale 10 de l'aiguille présente un biseau, de tenir compte de celui-ci dans le positionnement des séries de composants passifs. En effet, le biseau constitue une caractéristique physique de l'aiguille contribuant à une direction privilégiée de déformation lors de son insertion dans le tissu humain ou animal.

Comme on le voit sur les figures 3A et 3B, la génératrice G2 a été choisie correspondante à la pointe du biseau.

Dans le référentiel de la figure 3A, cette génératrice se projette en un point g2 sur l'axe Ox.

La génératrice G1 est choisie de manière à ce que l'angle non orienté (g1,O,g2) soit de 90°, g1 étant la projection orthogonale sur l'axe Oy de G1 dans le référentiel précédemment identifié.

Par ailleurs, dans cet exemple, les composants des deux séries 20 et 21 appartiennent deux à deux à un plan perpendiculaire à l'axe de l'aiguille.

Bien sûr, tout autre type de configuration peut être choisi sans pour autant sortir du cadre de la présente invention.

Ainsi, à titre d'exemple, on peut envisager de disposer les composants selon une ou plusieurs courbes hélicoïdales à la surface de l'aiguille, ou bien de manière aléatoire, sous réserve que les données (typiquement, les potentiels) issues de chaque série de composants permettent la détermination d'une relation biunivoque avec la position de l'aiguille par rapport à sa base, et/ou avec la position de son extrémité distale par rapport à sa base.

Il est également possible de tenir compte de la torsion de l'aiguille.

En effet, selon les tissus traversés, une aiguille chirurgical longue et fine, en plus des déformations « conventionnelles », est aussi susceptible de se déformer en torsion le long de son axe.

Le dispositif de l'invention permet de mesurer cette déformation en torsion en disposant, en plus des composants passifs précités, qui mesurent des déformations locales dans des plans contenant localement l'axe de l'aiguille, des composants passifs disposées de manière sensiblement orthogonale aux précédents de manière à mesurer des déformations locales dans des plans sensiblement orthogonaux audit axe de l'aiguille.

Les mesures issues de l'ensemble des composants passifs précités permettent donc de déterminer avec encore plus de précision la position de l'extrémité distale de l'aiguille.

### Fixation des composants

Les composants passifs peuvent être fixés sur l'aiguille par tout moyen approprié connu de l'homme du métier et compatible avec l'utilisation chirurgicale de l'aiguille.

Ils peuvent par exemple être collés à l'aide de colles spécifiques développées par les fabricants des composants, de préférence incorporés dans des logements ménagés dans la surface de l'aiguille de manière à ne pas dépasser de la surface de l'aiguille et ne pas interférer avec les tissus traversés, afin d'éviter tout risque de déplacement relatif des composants vis-à-vis de l'aiguille.

Toutefois, selon un mode de réalisation encore préféré qui va être décrit plus bas, les composants passifs sont intégrés à la paroi de l'aiguille au moyen de techniques de micro-fabrication.

### Connexion des composants et système électronique

Les informations individuelles de chaque composant passif peuvent être traitées soit de manière individuelle, soit de manière dite « intégrative » ou globale.

Dans le premier cas, chaque composant constitue à lui seul une série et est relié au système électronique qui traite les informations en provenance de chaque composant. Ce système permet de connaître les déformations de l'aiguille en chaque point où est positionné un capteur.

Dans le cadre d'un traitement « intégratif » des données, les composants d'une même série sont connectés en série, comme illustré à la figure 4.

Ainsi, lors de la déformation de l'aiguille, les composants au sein d'une même série sont amenés à se déformer, ce qui modifie leurs résistances respectives et par conséquent la résistance globale de la série.

Les variations de tension induites aux bornes de chaque série de composants reflètent les déplacements élémentaires de l'aiguille dans chacun des plans respectifs, orthogonaux à l'axe de l'aiguille tels que définis précédemment.

Après une étape de calibration, c'est-à-dire suite à l'établissement d'une relation univoque entre les déformations de l'aiguille et les données électriques mesurées par les composants passifs, il est possible de déterminer la position de l'extrémité distale de l'aiguille par rapport à son extrémité proximale à partir de la connaissance de l'information en tension.

Ainsi, à titre illustratif et en se reportant au référentiel orthogonal défini par les conditions des figures 3A et 3B, les déplacements Δx et Δy de l'extrémité distale de l'aiguille dans ce référentiel orthogonal sont déduits respectivement des informations de potentiels apportés par les séries de composants disposées respectivement le long des génératrices G1 et G2.

En outre, sous réserve d'une densité adaptée de composants le long des génératrices, il est aussi possible d'en déduire la déformation de l'ensemble de l'aiguille.

A cet effet, chaque série de composants est intégrée au sein d'un circuit électronique adapté, permettant l'acquisition des signaux électriques pertinents à partir desquels les informations spatiales recherchées (déformation et/ou position de l'extrémité distale de l'aiguille) sont déduites en vue d'être, par exemple, prises en compte dans un système de navigation.

Parmi les circuits électroniques appropriés, on peut citer par exemple le circuit TI XTR 106, qui est un circuit d'instrumentation prévu pour les mesures par jauges de contraintes.

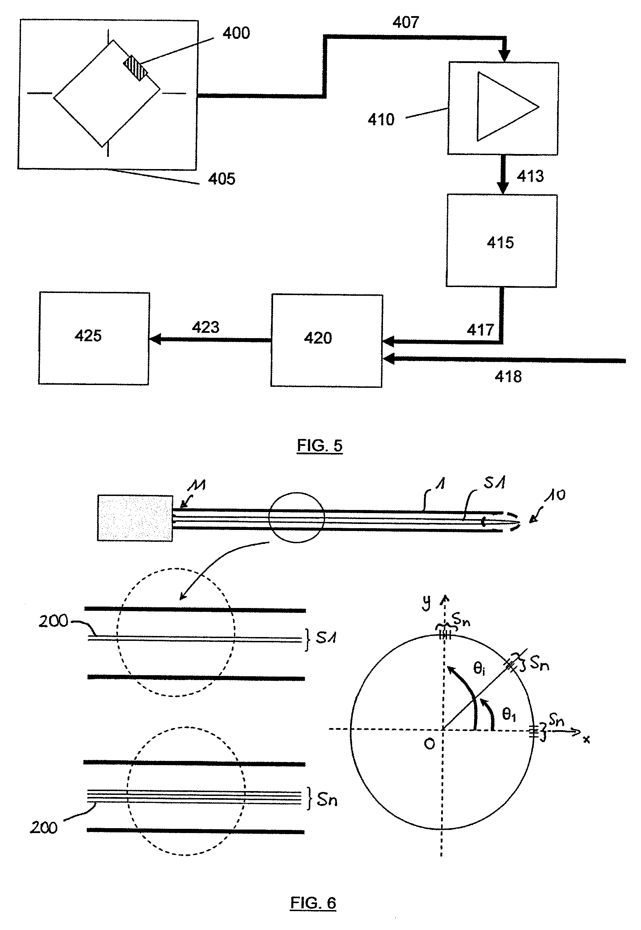

Le schéma de la figure 5 illustre un exemple de circuit électronique utilisé pour chaque série de composants.

La série 400 est intégrée à un pont de Wheatstone 405 permettant de mettre en évidence de faibles variations de résistance de l'ensemble.

La tension 407 de sortie du pont est alors amplifiée par un amplificateur 410 puis transférée à un dispositif 415 de linéarisation qui permet de linéariser la tension de sortie si nécessaire.

Le signal 417 de sortie est ensuite injecté au sein d'un microcontrôleur 420, ayant en particulier pour objet de transformer le signal selon un protocole de communication standardisé et interprétable par un ordinateur.

Ce signal 423 est alors traité au sein d'un ordinateur 425 et est interprété en fonction des informations provenant d'autres périphériques, comme par exemple un signal 418 linéarisé de tension d'une autre série de composant, des informations d'un système de navigation ou d'imagerie, etc.

De manière avantageuse, le système électronique peut présenter une fonction dite « offset » permettant de fixer à zéro, de manière automatique ou manuelle, la tension de sortie de chaque série de composants passifs lorsque l'aiguille n'est pas déformée.

### Incorporation des composants passifs à l'aiguille

Selon un aspect particulièrement avantageux de l'invention, les composants passifs ne sont pas des éléments rapportés sur l'aiguille, mais sont incorporés dans la surface de celui-ci grâce à des techniques de micro-fabrication.

On s'affranchit ainsi de tout risque de déplacement relatif des composants passifs par rapport à l'aiguille, ce qui permet d'assurer le caractère réellement biunivoque de la relation entre la position de l'aiguille ou de l'extrémité distale de celui-ci et les données issues des composants passifs.

Ce procédé implique typiquement des techniques de dépôt d'un élément conducteur à la surface de l'aiguille puis de gravure à travers un masque pour conférer à l'élément conducteur la forme souhaitée.

On pourra à cet égard se référer à l'article de L. Lin et al, « A Micro Strain Gauge with Mechanical Amplifier », Journal of Microelectromechanical Systems, Vol. 6, No. 4, Décembre 1997.

Il est ainsi possible d'incorporer l'élément conducteur sur toute la longueur de l'aiguille et selon des génératrices différentes.

En effet, la finesse et la précision des techniques de micro-fabrication permettent de réaliser, à la surface de l'aiguille, et selon la répartition angulaire souhaitée, une pluralité de jauges de contraintes chacune constituée d'un élément conducteur arrangé en formant au moins une spire.

Cette distribution permet, en particulier, d'améliorer la détection des déformations de l'aiguille.

La figure 6 illustre ainsi l'incorporation sur la surface de l'aiguille 1, d'un élément conducteur formant une unique spire S1 (i.e. un unique aller-retour du conducteur, de la base de l'aiguille à son extrémité), agencée sur toute la longueur de l'aiguille.

De même, afin d'augmenter la qualité de détection, un élément conducteur Sn comprenant un nombre n de spires longitudinales (allant de la base de l'aiguille à son extrémité) peut être agencé sur toute la longueur de l'aiguille.

Enfin, comme illustré sur la vue de droite de cette figure, qui est une vue en coupe de l'aiguille, plusieurs éléments conducteurs longitudinaux Sn peuvent être répartis sur la circonférence, chacun étant repérés par leur angle θ dans le système orthogonal de coordonnées polaires mentionné plus haut.

Dans ce cas, chaque élément conducteur S1 ou Sn est un composant passif constituant à lui seul une série.

A titre illustratif, dans le cas d'une déflexion de l'aiguille (telle qu'illustrée à la figure 1b), l'élément conducteur à potentiel maximal (et/ou minimal) permet de déterminer directement la direction angulaire de la déformation principale et donc la direction préférentielle de déplacement de l'extrémité distale de l'aiguille, à la résolution de la distribution angulaire près.

Cette configuration présente l'avantage de faciliter la gravure des différents éléments longitudinaux par répétition d'une pluralité d'étapes identiques.

A chaque étape de micro-fabrication, on peut ainsi réaliser une rotation de l'aiguille selon son axe d'un pas angulaire élémentaire.

D'autres configurations longitudinales ainsi que d'autres distributions angulaires sur tout ou partie de la circonférence de l'aiguille sont bien sûr envisageables.

En variante, il est possible d'incorporer non plus un système « intégratif » tel que celui de la figure 6 mais une série de systèmes élémentaires afin de mesurer les déformations locales puis d'en déduire une déformation globale.

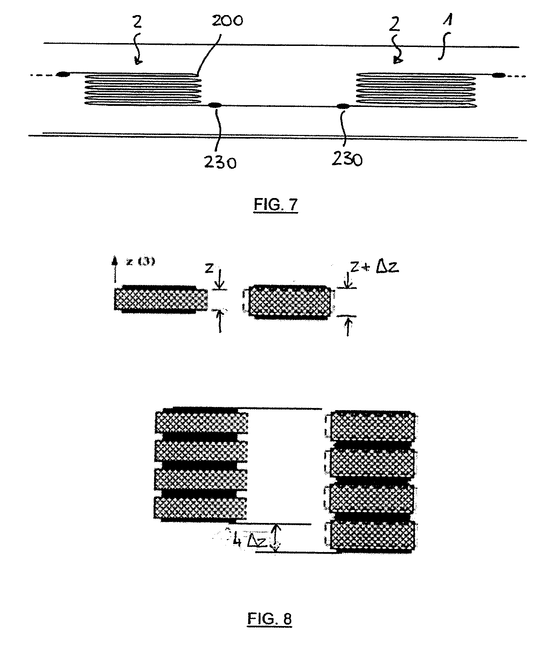

Un exemple est illustré à la figure 7.

Les techniques de micro-fabrication permettent en effet de développer un système miniaturisé et optimisé notamment en termes de connectique, laquelle est souvent un point faible voire limitant des systèmes « macroscopiques », du fait de sa fragilité et de son encombrement.

Comme on le voit sur la figure 7, les techniques de micro-fabrication de l'élément conducteur 200 permettent en effet de disposer les jauges 2 en série sur une génératrice avec les connecteurs 230 de deux jauges consécutives en vis-à-vis l'un de l'autre. Ceci constitue un avantage par rapport à la connexion des jauges telle qu'illustrée à la figure 4.

### Composants actifs

En plus du système de mesure des déformations, le dispositif peut comprendre un système dit « actif » de déformation, qui a pour objet d'appliquer, en des points prédéfinis de l'aiguille, des contraintes pour le déformer ou au contraire empêcher sa déformation.

Ceci permet de guider l'extrémité distale de l'aiguille vers la cible à atteindre, en évitant de porter atteinte à des organes situés sur sa trajectoire.

Ce système actif est composé d'un système mécanique et d'un système électronique.

Le système mécanique comprend une association de composants dits « actifs », situés sur une région de faible superficie de la surface de l'aiguille, et aptes à appliquer, sur cette région, une contrainte générant une déformation.

Les composants actifs peuvent reposer sur différents principes selon lesquels la fourniture d'une énergie entraîne une action mécanique. On peut ainsi envisager des microsystèmes électromécaniques (MEMS), et plus particulièrement des actionneurs piézoélectriques, thermiques, pneumatiques, électromagnétiques, électrostatiques, etc.

Comme les composants passifs décrits plus haut, les composants actifs sont fixés sur l'aiguille, dans des logements prévus à cet effet, ou bien, de manière préférée, incorporés dans la surface de celui-ci par des techniques de micro-fabrication.

Ils sont répartis selon au moins deux séries et sont disposés en des lieux de déformation privilégiée de l'aiguille.

Par exemple, les composants actifs de chaque série sont répartis selon deux génératrices distinctes de la surface de l'aiguille.

Dans le cas d'un actionneur piézo-électrique, l'application d'une tension permet d'allonger l'actionneur dans son logement, et donc d'appliquer des forces compressives sur les bords de ce logement, entraînant une rigidification ou au contraire une courbure de l'aiguille.

Les articles « Micro Mechatronics and Micro Actuators », Ishihara et al, IEEE/ASME Transactions on Mechatronics, Vol. 1, No. 1, Mars 2006 et « A Survey of Micro-Actuator Technologies for Future Spacecraft Missions » de Gilbertson et al, Journal of the British Interplanetary Society, Vol. 49, pp. 129-138, 1996, dressent des panoramas des différents modes d'actionnement utilisables.

Il va de soi que les composants actifs seront choisis par l'homme du métier en fonction de leurs performances.

Ainsi, dans la catégorie des actionneurs piézo-électriques, les actionneurs multicouches développent de grandes forces mais de faibles déflexions, tandis que les actionneurs bi-morphes ou multi-morphes engendrent de fortes déflexions mais de faibles forces.

La figure 8 illustre un actionneur multicouche constitué d'un empilement d'éléments circulaires. La sollicitation électrique d'un élément le fait se déformer en augmentant son épaisseur z d'une valeur Δz et en réduisant sa surface. L'épaisseur d'un empilement d'éléments circulaires subissant la même sollicitation augmente donc en proportion du nombre d'éléments empilés, ce qui permet une amplification de la déformation.

Par ailleurs, on peut utiliser les effets direct et inverse des matériaux piézo-électriques pour déterminer les déformations de l'aiguille et réaliser les actionneurs, pour autant que le composant permette d'imposer une déformation suffisante en tant qu'actionneur.

Selon une autre approche, les composants actifs sont des actionneurs thermiques disposés au sein de micro-articulations à base de polyimides, selon le concept présenté dans les articles de T. Ebefors et al, « New small radius joints based on thermal shrinkage of polyimide in V-grooves for robust self-assembly 3D microstructures », Micromech. Microeng. 8 (1998) 188-194 , « A walking silicon micro-robot », Transducers'99, 7-10 juin 1999, pp 1202-1205, et « A robust micro conveyer realized by arrayed polyimide joint actuators », J. Micromech. Microeng. 10 (2000) 337-349.

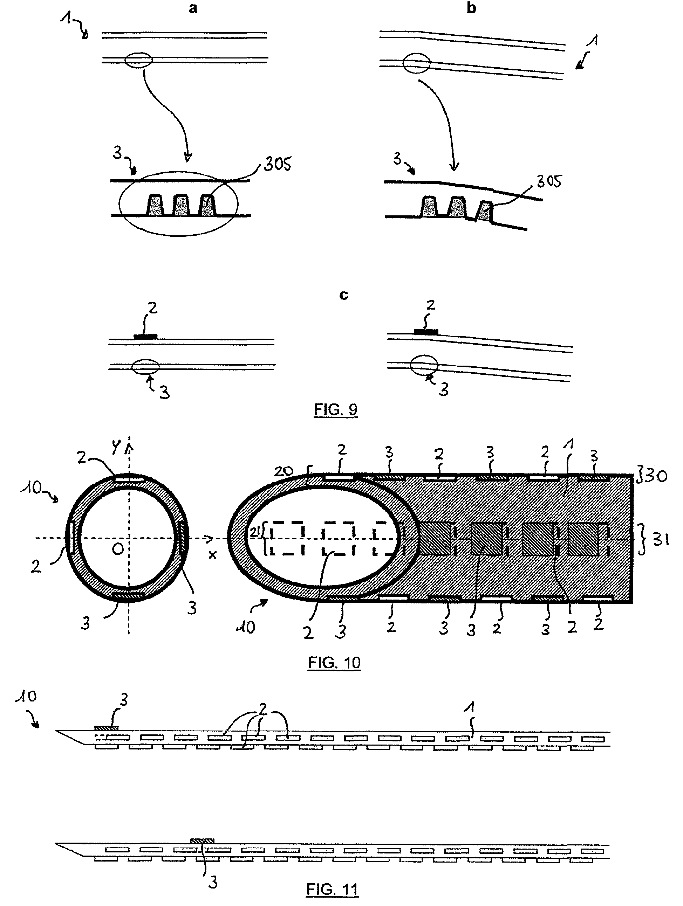

La figure 9 illustre un exemple basé sur l'emploi d'actionneurs thermiques 305 disposés dans des micro-articulations 3 à base de polyimide.

Le cas a correspond à la situation où le système est inactif, l'aiguille 1 n'étant pas déformée.

Lorsque la température locale augmente, l'actionneur 305 se contracte, ce qui déforme la micro-articulation 3 et modifie la courbure de l'aiguille. Cette situation est illustrée sur le schéma b. L'ensemble peut être recouvert d'une gaine isolante pour garantir la compatibilité biomédicale vis-à-vis de ces variations de température.

Ce type de micro-articulation peut être incorporé à la surface de l'aiguille au moyen de techniques de micro-fabrication.

Dans la variante illustrée sur le schéma c, des composants passifs 2 sont couplés aux composants actifs 3.

### Couplage des systèmes actifs et passifs

Selon un exemple non limitatif, illustré à la figure 10, l'aiguille est préférentiellement équipée à la fois de composants passifs tels que décrits précédemment, comme des jauges de contraintes, et de composants actifs. Dans l'exemple illustré, les composants actifs et passifs sont incorporés à la surface de l'aiguille au moyen des techniques de micro-fabrication évoquées plus haut.

De préférence, les séries 30, 31 de composants actifs 3 sont disposées selon deux génératrices diamétralement opposées à celles des deux séries 20, 21 de jauges 2.

Comme illustré à la figure 10, une alternance au niveau de chaque génératrice entre composants passif et actif est naturellement possible.

Un système de couplage du système passif et du système actif permet de constituer un dispositif dynamique dans lequel on ajuste, en temps réel, les contraintes à appliquer à l'aiguille en fonction de la position souhaitée, la résultante des contraintes étant évaluée grâce au système passif.

Ainsi, les actionneurs permettent, en fonction des informations issues des jauges, de corriger en temps réel la trajectoire de l'aiguille.

Ce couplage peut être effectué de manière globale ou au niveau des composants.

Ainsi, chaque composant passif peut être associé à un composant actif, comme illustré sur les figures 9c et 10.

Il n'est bien sûr pas impératif que les séries de composants passifs et de composants actifs présentent un agencement similaire.

Ainsi, en référence à la figure 11, un mode de réalisation particulièrement avantageux consiste à disposer des composants passifs sur toute la longueur de l'aiguille, par exemple selon différentes génératrices, et à positionner deux séries de composants actifs dans des zones de déformation préférentielle situées à proximité de l'extrémité distale de l'aiguille. Ce dispositif permet de mobiliser l'extrémité distale de l'aiguille tout en connaissant la déformation globale de celui-ci.

### Calibration du dispositif

Par calibration, on entend la détermination d'une correspondance (idéalement biunivoque) entre les informations acquises à l'aide des composants passifs positionnés sur l'aiguille ou les informations de commande des déformations exercées par les composants actifs positionnés sur l'aiguille, et les informations spatiales de l'aiguille déformée dans un référentiel lié rigidement à l'aiguille (ceci, quelles que soient les déformations de l'espace acceptable de déformation de l'aiguille).

En référence à la figure 1, on fixe à l'extrémité proximale 11 (ou base) de l'aiguille 1 un corps rigide C. L'aiguille est par ailleurs équipée des composants passifs et/ou actifs décrits plus haut.

Une première procédure de calibration, classique, permet de connaître, dans le référentiel Rₗ du localisateur, la position de la base 11 de l'aiguille et son référentiel R_{b} associé, quelles que soient les déformations de l'aiguille.

Lors d'une déformation de l'aiguille, chaque série de composants passifs est porteuse des informations intégrant les déformations locales au voisinage de chaque capteur de contraintes.

Une seconde procédure de calibration est nécessaire pour déterminer la correspondance biunivoque entre les informations de potentiels rapportées par les séries de composants et la position de l'extrémité distale 10 de l'aiguille par rapport à sa base 11, c'est-à-dire dans le référentiel R_{b}.

Par exemple, on peut calibrer une déformation simple de l'aiguille en déplaçant dans un plan l'extrémité distale de l'aiguille, au moyen d'un dispositif (robotisé ou non) de mobilisation micrométrique de l'extrémité distale de l'aiguille.

L'extrémité distale de l'aiguille peut être mobilisée par le dispositif micrométrique sur l'ensemble de l'espace acceptable de mobilisation de l'extrémité distale de l'aiguille (typiquement, une surface carrée), ou selon deux directions perpendiculaires simultanément ou bien successivement.

Les modalités de calibration dépendent, en particulier, des configurations géométriques adoptées pour les différentes séries de composants positionnés sur l'aiguille.

En effet, dans le cas où les séries de composants passifs ont été agencées de manière à définir un référentiel orthogonal (tel que présenté aux figures 3A et 3B), il n'est pas nécessaire d'effectuer la calibration pour tous les points de l'espace de mobilisation.

La détermination de cette relation biunivoque par exemple selon deux directions perpendiculaires de déplacement (voire quatre, pour une amélioration de la qualité de cette étape) permet de déduire, par interpolation, cette relation pour des déplacements dans toutes les directions.

A l'issue de cette procédure de calibration, il est possible de connaître la position de l'extrémité distale de l'aiguille dans le référentiel R_{b} associé à la base lors d'une déformation de l'aiguille. La position du référentiel R_{b} étant elle-même connue dans le référentiel Rₗ du localisateur, la position de l'extrémité de l'aiguille est ainsi connue dans RI.

Lorsque les composants de N séries sont disposés de manière aléatoire sur la surface de l'aiguille, la définition d'un référentiel est plus délicate et on s'assure qu'il est possible de déterminer une relation biunivoque entre les positions de l'extrémité distale de l'aiguille et les valeurs du N-uplet.

Dans le cas d'aiguilles jetables, la calibration est à effectuer pour chaque aiguille.

Dans le cas d'une aiguille dans laquelle les composants passifs ont été incorporés par les techniques de micro-fabrication évoquées plus haut, et ne sont donc pas susceptibles de se déplacer après l'intervention et la stérilisation, on peut considérer que la calibration reste valable lors d'utilisations successives de l'aiguille.

### Système de localisation

Les systèmes de localisation communément employés dans le cadre de procédure médicales naviguées, utilisant des techniques infrarouges, magnéto-optiques, etc. peuvent être employés en association avec le système de mesure et/ou d'application de déformations.

Ces systèmes reposent sur le principe de la triangulation qui permet de définir la position d'un point dans l'espace à partir de sa visualisation sous trois incidences différentes.

### Système d'imagerie

Le dispositif conforme à l'invention peut être utilisé en relation avec tous systèmes d'imagerie communément utilisés pour la visualisation de cibles et compatibles avec les composants utilisés.

De manière préférée, on utilise des imageries naviguées en temps réel, telles qu'une une imagerie échographique, imagerie vidéo fluorescente, entre autres.

Un système d'imagerie calibré permet de connaître à tout moment, par une recherche (« tracking ») de la cible visualisée sur l'image, la position de la cible dans le référentiel du localisateur.

Grâce au dispositif conforme à l'invention, la position de l'aiguille déformée, dans son ensemble, est connue dans le référentiel du localisateur.

La connaissance simultanée de la position de la cible et de l'aiguille déformée dans un même référentiel de l'environnement navigué permet ainsi de connaître la position relative de l'aiguille déformée et de son extrémité distale par rapport à la cible.

Il est important de préciser que, contrairement à de nombreuses procédures de l'art antérieur, il n'est pas nécessaire de visualiser l'aiguille déformée sur l'image permettant de voir la cible.

En effet, cette visualisation est déduite des informations acquises par les capteurs disposés le long de l'aiguille et à partir de la connaissance de la position du référentiel rigide lié à l'aiguille (corps rigide lié rigidement à la base de l'aiguille) dans le référentiel commun.

Lors de la réalisation du geste chirurgical, des contraintes peuvent être exercées en temps réel sur l'aiguille au moyen des composants actifs décrits plus haut, de manière à corriger au mieux la trajectoire de l'aiguille.

On peut ainsi imposer à l'aiguille d'aller en ligne droite vers la cible, ou bien d'emprunter une trajectoire planifiée compatible avec les degrés de mobilité et de déformation de l'aiguille.

Eventuellement, les composants actifs sont eux-mêmes calibrés, c'est-à-dire qu'on a défini au préalable une relation entre le potentiel imposé à une série de composants actifs, et la position de l'extrémité distale de l'aiguille par rapport à son extrémité proximale. Dans ce cas, l'emploi de composants passifs n'est pas indispensable, dans la mesure où, connaissant la position de la cible et celle de l'extrémité proximale de l'aiguille au moyen du système de localisation, le praticien peut imposer le potentiel nécessaire à chacune des séries de composants actifs pour obtenir la position voulue pour l'extrémité distale de l'aiguille.

Toutefois, il est également possible, lorsque les composants actifs sont couplés avec les composants passifs, de tenir compte des informations issues des composants passifs pour déterminer en temps réel le potentiel à appliquer aux composants actifs en vue d'obtenir la position de l'extrémité distale souhaitée. Le chirurgien procède par itérations mesures-application de contraintes.

Le dispositif conforme à l'invention trouve notamment application dans les procédures d'intervention chirurgicale robotisées.

Ainsi, le document WO03/094759 décrit un robot permettant de positionner et d'orienter une aiguille lors d'une intervention minimalement invasive. Ce robot est actionné par le chirurgien en vue d'imposer à l'aiguille une translation ou une rotation selon son axe.

En équipant l'aiguille des composants passifs et/ou actifs selon l'invention, on procure au chirurgien une aiguille « à tête chercheuse ».

Avec un tel outil, le chirurgien impose à l'aiguille ses mouvements de translation et/ou de rotation en direction de la cible, tandis que les systèmes passif et actif contrôlent la position de l'extrémité distale de l'aiguille par rapport à la cible.

Par ailleurs, le dispositif peut encore être enrichi de capteurs permettant d'évaluer la qualité des tissus (par exemple CMUT (acronyme du terme anglo-saxon « Capacitive Micromachined Ultrasonic Transducers ») et évaluation de la qualité tissulaire, fibre optique et analyse spectrale pour évaluation tissulaire, etc.).

### Avantages de l'invention

La calibration du dispositif permet une connaissance directe de la position de l'aiguille et/ou de son extrémité distale à partir de l'ensemble des signaux délivrés par les séries de capteurs passifs.

La possibilité de mieux prendre en compte les déformations de l'aiguille-voire de les influencer - permet d'améliorer la précision des interventions percutanées à l'aide de l'aiguille.

Ceci a pour conséquence directe de pouvoir diminuer le nombre d'essais nécessaires à la réalisation de ces gestes et donc, de manière plus générale, leurs morbidités. Il sera aussi probablement possible d'envisager la réalisation de nouveaux gestes, non réalisés jusqu'à ce jour car non envisageables avec les outils actuels du fait des risques inhérents à leur réalisation.

L'amélioration de la possibilité de guidage de ces aiguilles permet la réalisation des procédures profondes avec des aiguilles de plus petit diamètre (l'augmentation de diamètre de l'aiguille est un moyen de s'affranchir des déformations de ce dernier) et donc moins invasifs.

Par ailleurs, l'emploi de techniques de micro-fabrication permet d'incorporer les composants passifs et/ou actifs dans la surface de l'aiguille, ce qui permet de s'affranchir des contraintes liées aux modes de fixation (colle, gaine...) des composants classiques, mais aussi des contraintes liées à l'encombrement électronique, permettant ainsi d'avoir un outil réellement compatible avec la clinique.

Enfin, le dispositif ne s'applique pas qu'à des interventions de ponction ou de biopsie, mais trouve des applications en chirurgie minimalement invasive.

A titre d'exemple, la prise en compte de déformations d'un palpeur lors d'une intervention orthopédique naviguée permettrait d'améliorer la qualité lors d'une intervention orthopédique naviguée permettrait d'améliorer la qualité de localisation de la zone palpée. Dans le cadre de chirurgies laparoscopiques, les instruments manipulés et équipés du système développé offriraient la possibilité de les naviguer de manière « naturelle » dans un référentiel commun.

## Revendications

1. Dispositif d'intervention chirurgicale, comprenant un instrument chirurgical (1) apte à traverser des tissus humains ou animaux, ledit instrument étant une aiguille, et un système comprenant des composants dits « passifs » aptes à mesurer une déformation ou une contrainte locale de l'instrument, **caractérisé en ce que** ledit système comprend au moins deux séries (20, 21) de composants passifs agencés à la surface de l'instrument de manière à établir une relation biunivoque entre les informations spatiales de l'instrument dans un référentiel rigidement lié audit instrument et l'ensemble des données issues des dites séries.

2. Dispositif selon la revendication 1, **caractérisé en ce que** ledit système comprend au moins deux séries (20, 21) de composants passifs (2) disposés selon deux génératrices de la surface de l'instrument appartenant à deux plans non confondus passant par l'axe de l'instrument, définissant ainsi un référentiel (O,x,y) dans un plan orthogonal à l'axe de l'instrument.

3. Dispositif selon la revendication 2, **caractérisé en ce que** lesdits composants passifs (2) comprennent des microsystèmes électromécaniques, tels que des capteurs piézoélectriques et/ou des jauges de contraintes.

4. Dispositif selon l'une des revendications 2 à 3, **caractérisé en ce que** les composants passifs sont incorporés dans la surface de l'instrument.

5. Dispositif selon la revendication 4, **caractérisé en ce que** chaque série (20, 21) comprend un unique composant passif, lequel comporte un élément conducteur (S1, Sn) longitudinal, se présentant sous la forme d'au moins une spire, s'étendant parallèlement à l'axe de l'instrument sur sensiblement toute la longueur de l'instrument.

6. Dispositif selon l'une des revendications 1 à 5, **caractérisé en ce que** l'instrument présente au moins une zone déformable, telle qu'une diminution locale d'épaisseur et/ou une articulation, et **en ce que** les composants passifs sont agencés sur ladite zone.

7. Dispositif selon l'une des revendications 1 à 6, **caractérisé en ce que** le système comprend en outre au moins deux séries (30, 31) de composants (3) dits « actifs » aptes à imposer une contrainte locale à l'instrument agencés à la surface de l'instrument.

8. Dispositif selon la revendication 7, **caractérisé en ce que** ledit système comprend au moins deux séries (30, 31) de composants actifs (3) agencés sur au moins une zone de déformation préférentielle de l'instrument.

9. Dispositif selon la revendication 8, **caractérisé en ce que** lesdits composants actifs (3) comprennent des microsystèmes électromécaniques, tels que des actionneurs thermiques, piézoélectriques, pneumatiques, électromagnétiques, et/ou électrostatiques.

10. Dispositif selon l'une des revendications 8 ou 9, **caractérisé en ce que** les composants actifs (3) sont incorporés dans la surface de l'instrument (1).

11. Dispositif selon l'une des revendications 1 à 10, **caractérisé en ce que** ledit système comprend au moins deux séries (20, 21) de composants passifs (2) et deux séries (30, 31) de composants actifs (3).

12. Dispositif selon la revendication 11, **caractérisé en ce que** les composants passifs (2) de chaque série sont répartis sensiblement sur toute la longueur de l'instrument chirurgical (1) et/ou **en ce que** les composants actifs sont agencés sur au moins une zone de déformation préférentielle de la partie distale de l'instrument.

13. Dispositif selon l'une des revendications 11 ou 12, **caractérisé en ce qu'**il comprend un système de couplage des composants passifs et actifs.

14. Dispositif selon l'une des revendications 1 à 13, **caractérisé en ce que** lesdites informations spatiales comprennent la position de l'extrémité distale de l'instrument par rapport à l'extrémité proximale.

15. Procédé de fabrication d'un dispositif conforme à l'une des revendications 1 à 14, **caractérisé en ce qu'**il comprend l'incorporation, dans la surface de l'instrument, des composants passifs et, le cas échéant, des composants actifs, au moyen de techniques de micro-fabrication.

## Patentansprüche

1. Vorrichtung für chirurgischen Eingriff, umfassend ein chirurgisches Instrument (1), das imstande ist, Gewebe von Mensch oder Tier zu durchdringen, wobei das Instrument eine Nadel ist, und ein System, das sogenannte "passive" Komponenten umfasst, die imstande sind, eine Verformung oder eine lokale Spannung des Instruments zu messen, **dadurch gekennzeichnet, dass** das System mindestens zwei Reihen (20, 21) passive Komponenten umfasst, die auf der Oberfläche des Instruments derart ausgebildet sind, dass eine eineindeutige Beziehung zwischen den räumlichen Informationen des Instruments in einem Bezugssystem, das starr mit dem Instrument verbunden ist, und der Gruppe von Daten, die von den Reihen ausgegeben werden, hergestellt wird.

2. Vorrichtung nach Anspruch 1, **dadurch gekennzeichnet, dass** das System mindestens zwei Reihen (20, 21) passive Komponenten (2) umfasst, die gemäß zwei Mantellinien der Oberfläche des Instruments, die zu zwei nicht zusammenfallenden Ebenen gehören, die durch die Achse des Instruments verlaufen, angeordnet sind, so dass ein Bezugssystem (O,x,y) in einer zur Achse des Instruments orthogonalen Ebene gebildet wird.

3. Vorrichtung nach Anspruch 2, **dadurch gekennzeichnet, dass** die passiven Komponenten (2) elektromechanische Mikrosysteme wie piezoelektrische Sensoren und/oder Spannungsfühler umfassen.

4. Vorrichtung nach einem der Ansprüche 2 bis 3, **dadurch gekennzeichnet, dass** die passiven Komponenten in die Oberfläche des Instruments eingearbeitet sind.

5. Vorrichtung nach Anspruch 4, **dadurch gekennzeichnet, dass** jede Reihe (20, 21) eine einzige passive Komponente umfasst, welche ein längliches leitendes Element (S1, Sn) in Form von mindestens einer Windung aufweist, das sich parallel zur Achse des Instruments über etwa die gesamte Länge des Instruments erstreckt.

6. Vorrichtung nach einem der Ansprüche 1 bis 5, **dadurch gekennzeichnet, dass** das Instrument mindestens eine verformbare Zone wie eine lokale Dickenreduzierung und/oder ein Gelenk aufweist, und dass die passiven Komponenten auf dieser Zone ausgebildet sind.

7. Vorrichtung nach einem der Ansprüche 1 bis 6, **dadurch gekennzeichnet, dass** das System ferner mindestens zwei Reihen (30, 31) sogenannter "aktiver" Komponenten (3) umfasst, die imstande sind, auf das Instrument eine lokale Spannung auszuüben, die auf der Oberfläche des Instruments ausgebildet sind.

8. Vorrichtung nach Anspruch 7, **dadurch gekennzeichnet, dass** das System mindestens zwei Reihen (30, 31) aktive Komponenten (3) umfasst, die auf mindestens einer bevorzugten Verformungszone des Instruments ausgebildet sind.

9. Vorrichtung nach Anspruch 8, **dadurch gekennzeichnet, dass** die aktiven Komponenten (3) elektromechanische Mikrosysteme wie thermische, piezoelektrische, pneumatische, elektromagnetische und/oder elektrostatische Aktuatoren umfassen.

10. Vorrichtung nach einem der Ansprüche 8 oder 9, **dadurch gekennzeichnet, dass** die aktiven Komponenten (3) in die Oberfläche des Instruments (1) eingearbeitet sind.

11. Vorrichtung nach einem der Ansprüche 1 bis 10, **dadurch gekennzeichnet, dass** das System mindestens zwei Reihen (20, 21) passive Komponenten (2) und zwei Reihen (30, 31) aktive Komponenten (3) umfasst.

12. Vorrichtung nach Anspruch 11, **dadurch gekennzeichnet, dass** die passiven Komponenten (2) jeder Reihe über etwa die gesamte Länge des chirurgischen Instruments (1) verteilt sind und/oder dass die aktiven Komponenten auf mindestens einer bevorzugten Verformungszone des distalen Teils des Instruments ausgebildet sind.

13. Vorrichtung nach einem der Ansprüche 11 oder 12, **dadurch gekennzeichnet, dass** sie ein Kopplungssystem der passiven und aktiven Komponenten umfasst.

14. Vorrichtung nach einem der Ansprüche 1 bis 13, **dadurch gekennzeichnet, dass** die räumlichen Informationen die Position des distalen Endes des Instruments in Bezug auf das proximale Ende umfassen.

15. Herstellungsverfahren einer Vorrichtung nach einem der Ansprüche 1 bis 14, **dadurch gekennzeichnet, dass** es die Einarbeitung in die Oberfläche des Instruments der passiven Komponenten und gegebenenfalls der aktiven Komponenten mit Hilfe von Techniken der Mikrofabrikation umfasst.

## Claims

1. A surgical intervention device, comprising a surgical instrument (1) capable of passing through human or animal tissue, wherein said instrument is a needle, and a system comprising so-called "passive" components capable of measuring a deformation or a local strain of the instrument, **characterized in that** said system comprises at least two series (20, 21) of passive components arranged at the surface of the instrument so as to set up a biunivocal relation between the spatial information of the instrument in a referential linked rigidly to said instrument and all the data issuing from said series.

2. The device according to claim 1, **characterized in that** said system comprises at least two series (20, 21) of passive components (2) arranged according to two generatrices of the surface of the instrument belonging to two not combined planes passing through the axis of the instrument, defining a referential (O,x,y) in a plane orthogonal to the axis of the instrument.

3. The device according to claim 2, **characterized in that** said passive components (2) comprise electromechanical microsystems, such as piezoelectric sensors and/or strain gauges.

4. The device according to one of claims 2 to 3, **characterized in that** the passive components are incorporated into the surface of the instrument.

5. The device according to claim 4, **characterized in that** each series (20, 21) comprises a single passive component which comprises a longitudinal conductive element (S1, Sn) in the form of at least one coil, extending parallel to the axis of the instrument over substantially the entire length of the instrument.

6. The device according to one of claims 1 to 5, **characterized in that** the instrument has at least one deformable zone, such as a decrease in local thickness and/or articulation, and **in that** the passive components are arranged on said zone.

7. The device according to one of claims 1 to 6, **characterized in that** said system further comprises at least two series (30, 31) of so-called "active" components (3) capable of imposing a local strain on the instrument arranged at the surface of the instrument.

8. The device according to claim 7, **characterized in that** said system comprises at least two series (30, 31) of active components (3) arranged on at least one preferential deformation zone of the instrument.

9. The device according to claim 8, **characterized in that** said active components (3) comprise electromechanical microsystems, such as thermal, piezoelectric, pneumatic, electromagnetic, and/or electrostatic actuators.

10. The device according to one of claims 8 or 9, **characterized in that** the active components (3) are incorporated into the surface of the instrument (1).

11. The device according to one of claims 1 to 10, **characterized in that** said system comprises at least two series (20, 21) of passive components (2) and two series (30, 31) of active components (3).

12. The device according to claim 11, **characterized in that** the passive components (2) of each series are distributed substantially over the entire length of the surgical instrument (1) and/or **in that** the active components are arranged on at least one preferential deformation zone of the distal part of the instrument.

13. The device according to one of claims 11 or 12, **characterized in that** it comprises a coupling system of the passive and active components.

14. The device according to one of claims 1 to 13, **characterized in that** said spatial information comprise the position of the distal end of the instrument relative to the proximal end.

15. A production process of a device according to one of claims 1 to 14, **characterized in that** it comprises incorporating in the surface of the instrument, passive components and, where required, active components, by means of micro-production techniques.
